# EUROPEAN PATENT SPECIFICATION

(11) **EP 3 533 423 B1**
(45) Date of publication and mention of the grant of the patent: **07.06.2023**
(21) Application number: 19159344.1
(22) Date of filing: 26.02.2019
(51) Int. Cl.: A61F 5/01, A61F 5/058

(54) **HIGH-ARCHED FOOT ORTHOSIS**
HOHLFUSSSCHUHEINLAGE
ORTHÈSE DE PIED FORTEMENT ARQUÉE

(30) Priority: 02.03.2018 TW 107107059
(43) Date of publication of application: 04.09.2019
(73) Proprietor: Hsu, Wen Hua, Kaohsiung City (TW)
(72) Inventor: Hsu, Wen Hua, Kaohsiung City (TW)
(74) Representative: Pallini Gervasi, Diego

(56) References cited:
- WO-A1-2012/076398
- GB-A- 2 330 309
- JP-U- H0 595 516
- US-A1- 2015 320 581

## Description

### 1. Field of the Invention

The present invention relates to an orthosis for lower limbs, and more particularly to a high-arched foot orthosis that may correct supination of foot (high-arched foot), external rotation of lower limbs, knee varus, and pelvic tilt and torsion of a wearer.

### 2. Description of Related Art

Some people are born with foot supination, which is also known as high-arched foot (the medial arches of the person are highly arched), and talus abduction combine eversion. An ankle joint and an above-located subtalar joint of the high-arched foot suffer subluxation. External rotations of a tibia and a femur cause knee varus. Furthermore, an acetabulum of a pelvis is pushed by a femoral head of the externally rotating femur, and results in pelvic tilt. The spine will then suffer curved spine compensation due to the tilting pelvis and cause functional scoliosis. Tensions of paraspine muscles and soft tissues of lower limbs of a human body may also be unbalanced.

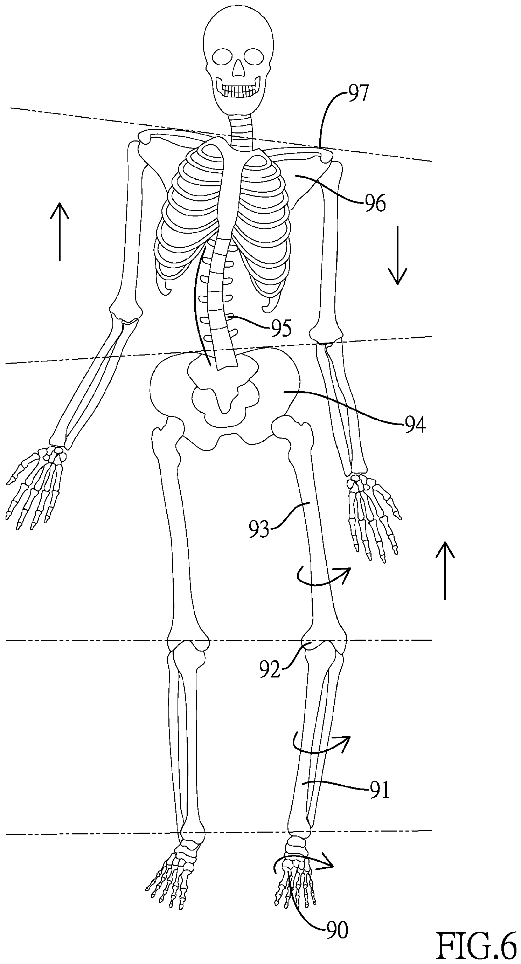

With reference to Fig. 6, one foot 90 of a person suffers hypersupination, and in consequence a tibia 91 and a femur 93 of the person rotate externally. A knee joint of the person is thereby varus, which causes bowed legs 92. External rotation of the femur 93 forces a femoral head thereof to move forwardly, which further pushes an acetabulum of a pelvis 94. As a result, the pelvis 94 becomes tilted. Accordingly, the spine 95 of the person becomes curved spine compensation by the pelvis 94, and tilts a scapula 96 and a clavicle 97 of the person. In a long term, such disorder and inadequate bone and joint alignments will lead to harmful postures to the person, and a series of symptoms may accordingly occur, such as: Achilles tendon pain, shin splints, LCL (lateral collateral ligament) stretch and medial meniscus compression, femoral acetabular impingement, gluteus medius dysfunction, pelvic tilt and torsion, functional leg length inequality, functional scoliosis, psoas major & multifidus stretch and weakness, SI (sacroiliac) joint subluxation & sacroiliitis, HIVD (Herniation of Inter-Vertebral Disc), lumbar facet joint syndrome, soft tissue tightness, spondylolithesis, shoulder elevation compensation dysfunction, CT (cervical thoracic) junction, cervical disc herniation, cervical facet joint syndrome, neck and upper back myofacial problem, TMJ (temporomandibular joint) subluxation, unilateral headache, etc.

JP H05 95516 U is considered to represent the closest prior art, it discloses a foot covering with ankle straps. The foot covering with ankle straps has a sock-like body and four bundle bands attaching to the body by sewing, weaving, or adhesion. The four bundle bands may fix the ankle joint and tarsal bones of a wearer's foot, so that the foot may only move in a uniaxial spiral direction. Although JP H05 95516 U may prevent or correct a flat foot, it cannot help with other severer symptoms such as knee valgus and scoliosis, and it disclosed nothing for correcting a high-arched foot, which needs a corrective force in a totally opposite direction.

The main objective of the present invention is to provide a high-arched foot orthosis that may correct the supinated (high-arched) foot, and improve bone and joint alignments and proper biomechanics. Thus the wearer's lower limbs may be kept in correct bone and joint alignments, and poor postures, and subsequent discomfort and muscular pains may be reduced.

The high-arched foot orthosis in accordance with the present invention comprises two bodies, and each one of the two bodies has a wearing space, a foot portion, an ankle portion, a shank portion, a substrate, and an elastomer. The foot portion has a sole located on a bottom of the foot portion, an instep located on a top of the foot portion, an arch, and a lateral side. The arch and the lateral side are located on two vertical sides of the foot portion, wherein the arch is located on one of the two vertical sides facing the other one of the two bodies. Each one of the arch and the lateral side is connected to the sole and the instep. The ankle portion is connected to the foot portion. The shank portion is connected to the ankle portion. The substrate and the elastomer are disposed adjacent to each other, and surround the wearing space. The substrate extends from the foot portion to the shank portion through the ankle portion. The elastomer extends from the instep of the foot portion at a cuneiform bone of a wearer through the arch and the sole in order, wraps over a cuboid bone of the wearer, extends through the ankle portion inwardly and upwardly toward the shank portion, and wraps over a talus bone of the wearer.

Other objectives, advantages and novel features of the invention will be described in the following detailed description when taken in conjunction with the accompanying drawings.

### IN THE DRAWINGS:

Fig. 1 is a front side view of a first embodiment of a high-arched foot orthosis
Fig. 2 is a front side view of a second embodiment of a high-arched foot orthosis in accordance with the present invention;
Fig. 3 is a rear side view of the high-arched foot orthosis in Fig. 2;
Fig. 4 is a front side view of a third embodiment of a high-arched foot orthosis in accordance with the present invention;
Fig. 5 is a rear side view of the high-arched foot orthosis in Fig. 4; and
Fig. 6 shows a supinated foot of a person and disorder of legs and a human body of the person that is caused by the supinated foot.

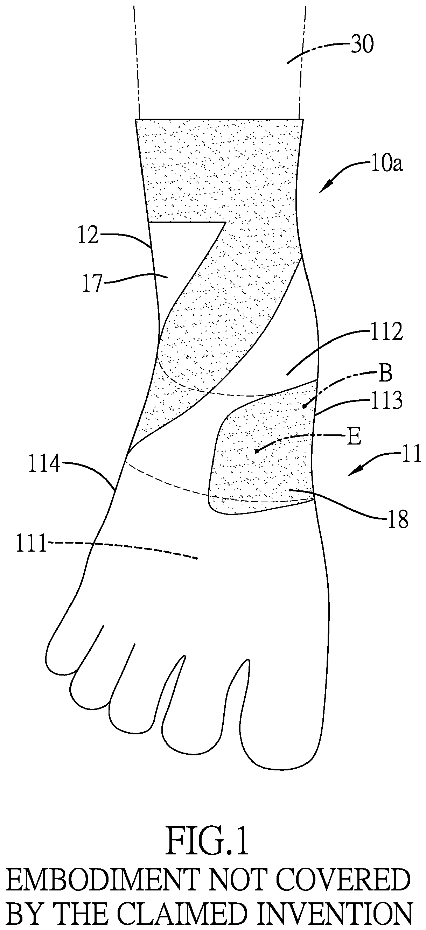

With reference to Figs. 1, 2, and 4, the present disclosure provides three embodiments of a high-arched foot orthosis, wherein the first embodiment and Fig. 1 are not encompassed by the claimed invention.

The scope of the invention is solely defined by the appended claims.

In either one of the three embodiments, the high-arched foot orthosis comprises two bodies 10a, 10b, 10c. Each one of the two bodies 10a, 10b, 10c has a wearing space disposed therein, and has a foot portion 11 and an ankle portion 12. The foot portion 11 comprises a sole 111, an instep 112, an arch 113, and a lateral side 114. The sole 111 is located on a bottom of the foot portion 11.The instep 112 is located on a top of the foot portion 11. The sole 111 and the instep 112 are vertically aligned with each other. The arch 113 and the lateral side 114 are located on two vertical sides of the foot portion 11, wherein the arch 113 is on one of the two vertical sides that faces the other one of the two bodies 10a, 10b, 10c. Each one of the arch 113 and the lateral side 114 is connected with the sole 111 and the instep 112. The ankle portion 12 is connected to the foot portion 11.

Each one of the two bodies 10a, 10b, 10c comprises a substrate 17 and an elastomer 18. The substrate 17 and the elastomer 18 are disposed adjacent to each other, and surround the wearing space of the body 10a, 10b, 10c. The substrate 17 extends from the foot portion 11 to the ankle portion 12. The elastomer 18 extends from the foot portion 11, passes through the arch 113, the sole 111, and the lateral side 114 in order, and extends toward the ankle portion 12.

With reference to Figs. 1, 2, and 4, the elastomer 18 wraps over a cuneiform bone E of a wearer 30 at the instep 112, and extends through the arch 113, the sole 111, and the lateral side 114 in order. The elastomer 18 wraps over a navicular bone B of the wearer 30. Afterwards, the elastomer 18 wraps over a cuboid bone A of the wearer 30 at the lateral side 114, spirally extends to the ankle portion 12, and wraps over a talus bone C of the wearer 30 at the ankle portion 12.

With reference to Fig. 1, in a first embodiment, the high-arched foot orthosis is configured as a sock. In each one of the two bodies 10a, the ankle portion 12 has a first opening disposed at an upper end of the ankle portion 12. The first opening of the ankle portion 12 communicates with the wearing space of the body 10a so that the wearer 30 may put on the body 10a. The elastomer 18 surrounds and fetters a shank portion that is connected to the ankle portion 12. The elastomer 18 extends from the instep 112 near the arch 113, wraps over the cuneiform bone E, passes through the arch 113, the sole 111, and the lateral side 114, and extends to the ankle portion 12 from exterior to interior. Thereby the elastomer 18 provides an inner combine downward pressing force on the wearer's 30 foot arch to correct the supinated foot of the wearer 30.

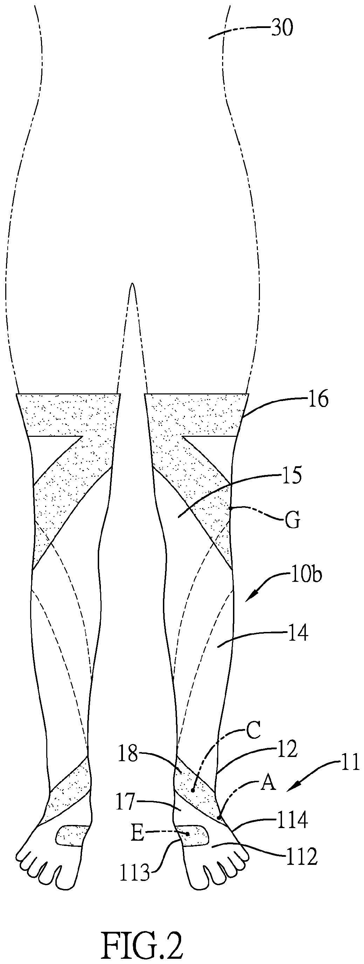

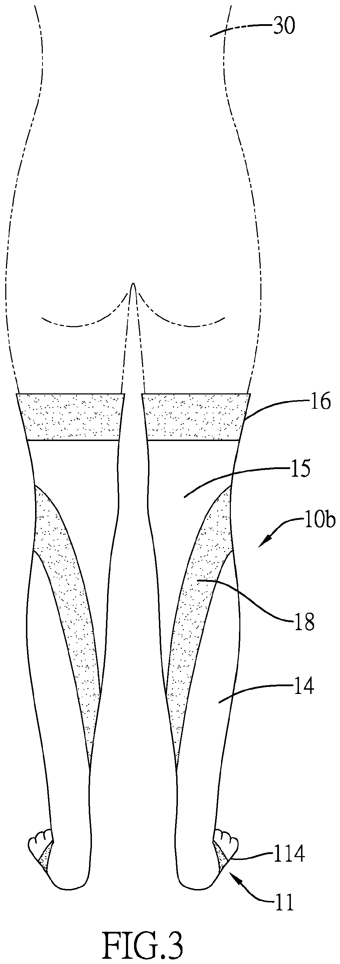

With reference to Figs. 2 and 3, in a second embodiment, the high-arched foot orthosis is configured as an over-the-knee sock. Each one of the two bodies 10b comprises a shank portion 14, a knee portion 15, and a thigh portion 16. A lower end of the shank portion 14 is connected to an upper end of the ankle portion 12. The knee portion 15 is connected to an upper end of the shank portion 14. The thigh portion 16 is connected to the knee portion 15, and is connected to the shank portion 14 through the knee portion 15. The substrate 17 extends from the foot portion 11 to the thigh portion 16 through the ankle portion 12, the shank portion 14, and the knee portion 15 in order. The thigh portion 16 has a second opening communicating with the wearing space of the body 10b so that the wearer 30 may put on the body 10b. Based on the first embodiment, with reference to Figs. 2 and 3, the elastomer 18 extends from the ankle portion 12 through a rear side of the shank portion 14, obliquely extends in combination with upward and outward turns through an outer side of the knee portion 15, and wraps over a fibula head G and an outer side of a knee joint of the wearer 30. The elastomer 18 eventually extends to a front side of the thigh portion 16, and surrounds and fetters on the thigh portion 16.

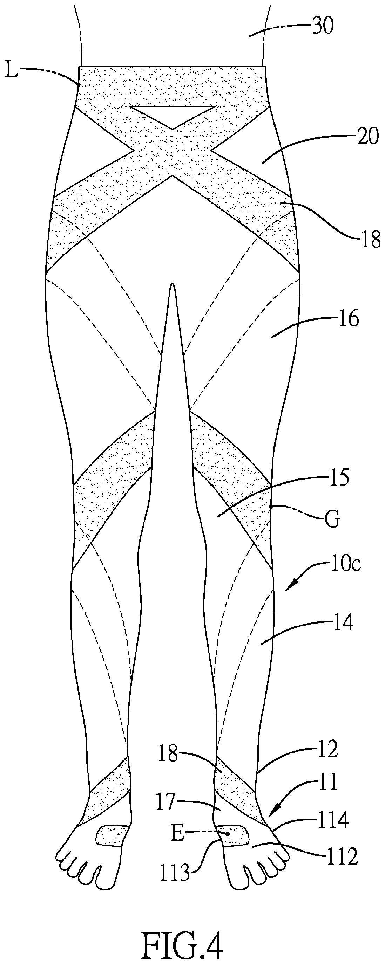

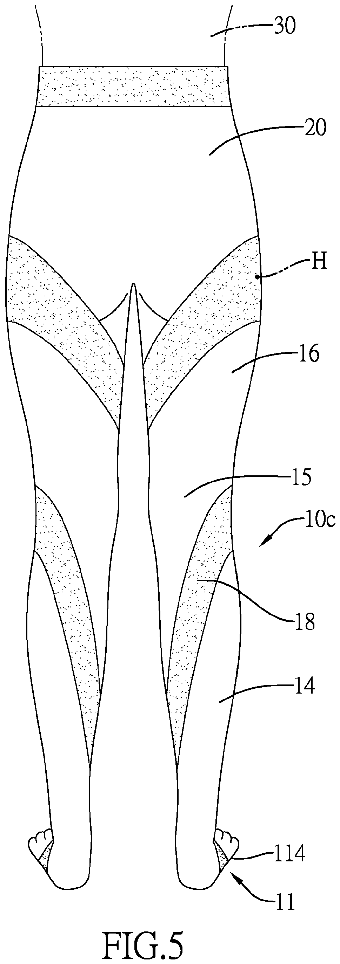

Furthermore, with reference to Figs. 4 and 5, in a third embodiment, the high-arched foot orthosis is configured as a pantyhose. Each one of the two bodies 10c comprises a shank portion 14, a knee portion 15, and a thigh portion 16. A lower end of the shank portion 14 is connected to an upper end of the ankle portion 12. The knee portion 15 is connected to an upper end of the shank portion 14. The thigh portion 16 is connected to the knee portion 15, and is connected to the shank portion 14 through the knee portion 15. The substrate 17 extends from the foot portion 11 through the ankle portion 12, the shank portion 14, the knee portion 15, and the thigh portion 16 in order.

Moreover, in the third embodiment, the high-arched foot orthosis comprises a pelvis portion 20. The pelvis portion 20 is connected to upper ends of the two thigh portions 16 of the two bodies 10c. The pelvis portion 20 has a third opening communicating with the wearing spaces of the two bodies 10c so that the wearer 30 may put on the high-arched foot orthosis. Based on the second embodiment, with reference to Figs. 4 and 5, the elastomer 18 of each one of the two bodies 10c extends through a rear side of the shank portion 14, obliquely extends in combination with upward and outward turns through an outer side of the knee portion 15, and wraps over a fibula head G and an outer side of a knee joint of the wearer 30. Afterwards the elastomer 18 spirally extends upwardly through a front side and a rear side of the thigh portion 16, and wraps over a hip joint H of the wearer 30. The elastomer 18 further extends toward a front side of the pelvis portion 20. The elastomers 18 of the two bodies 10c intersect with each other and form an X-shape at the front side of the pelvis portion 20. Finally, wrapping over an iliac crest L of the wearer 30, the elastomer 18 surrounds and fetters where the wearer's pelvis and the wearer's waist meet.

In previous descriptions, all parts of the high-arched foot orthosis, including the elastomer 18 of each one of the two bodies 10a, 10b, 10c, are adequately elastic and tensile.

With reference to Figs. 2 and 3, in the second embodiment, the elastomer 18 extends through the shank portion 14 and fetters the thigh portion 16, so the elastomer 18 may provide a torque to internally rotate the wearer's shank and thigh. Also the elastomer 18 may provide an inward pushing force on the wearer's knee. Therefore, the knee varus of the wearer 30 may be corrected by the high-arched foot orthosis.

With reference to Figs. 4 and 5, based on the second embodiment, the high-arched foot orthosis in accordance with the third embodiment may also correct knee varus. Furthermore, because the elastomer 18 extends to the pelvis portion 20 through the thigh portion 16, the elastomer 18 may force the wearer's hip joint H to rotate forwardly and inwardly, and thereby the external rotation of the femur and the pelvic tilt may be corrected.

With the aforementioned technical characteristics, the high-arched foot orthosis utilizes each one of the elastomers 18 to press a corresponding arch 113, further to push a corresponding knee portion 15, or to rotate the pelvis portion 20 by applying corrective forces to relevant parts of the wearer 30. Therefore, correct bone and joint alignments and proper biomechanics may be kept for the wearer 30. Poor postures, and subsequent discomfort, and muscular sores & pains due to disordered bone and joint alignments may be alleviated. Once the high-arched foot has been corrected by the present invention, health and comfort will improve on the wearer 30.

When the wearer 30 wears the high-arched foot orthosis, the stretching and pressing forces provided by the elastomer 18 may correct bone and joint alignments of the wearer's feet, lower limbs, knee joints, hip joint H, and pelvis, and prevent high-arched medial arches of feet, knee varus, and pelvic tilt and torsion.

## Claims

1. A high-arched foot orthosis adapted to be applied onto lower limbs of a human body, and comprising:
two bodies (10b), each one of the two bodies (10b) having a wearing space formed inside the body (10b);
a foot portion (11) having
a sole (111) located on a bottom of the foot portion (11);
an instep (112) located on a top of the foot portion (11);
an arch (113) located on one of two vertical sides of the foot portion (11), facing the other one of the two bodies (10b), and connected to the sole (111) and the instep (112); and
a lateral side (114) located on the other one of the two vertical sides of the foot portion (11), and connected to the sole (111) and the instep (112);
an ankle portion (12) connected to the foot portion (11);
a shank portion (14) connected to the ankle portion (12);
a substrate (17) surrounding the wearing space and extending from the foot portion (11) to the shank portion (14) through the ankle portion (12); and an elastomer (18) disposed adjacent to the substrate (17) and surrounding the wearing space, and when in use, extending from the instep (112) of the foot portion (11) at a cuneiform bone (E) of a wearer (30) through the arch (113) and the sole (111) in order, wrapping over a cuboid bone (A) of the wearer (30), extending through the ankle portion (12) inwardly and upwardly toward the shank portion (14), and wrapping over a talus bone (C) of the wearer (30); and **characterized in that**
each one of the two bodies (10b) has
a knee portion (15) connected to the shank portion (14); and
a thigh portion (16) connected to the knee portion (15);
wherein the elastomer (18) of each one of the two bodies (10b) extends to the thigh portion (16) through a rear side of the shank portion (14), obliquely extends in combination with upward and outward turns through an outer side of the knee portion (15) toward a front side of the thigh portion (16), and surrounds and fetters the thigh portion (16).

2. A high-arched foot orthosis adapted to be applied onto lower limbs of a human body, and comprising:
two bodies (10c), each one of the two bodies (10c) having
a wearing space formed inside the body (10c);
a foot portion (11) having
a sole (111) located on a bottom of the foot portion (11);
an instep (112) located on a top of the foot portion (11);
an arch (113) located on one of two vertical sides of the foot portion (11), facing the other one of the two bodies (10c), and connected to the sole (111) and the instep (112); and
a lateral side (114) located on the other one of the two vertical sides of the foot portion (11), and connected to the sole (111) and the instep (112);
an ankle portion (12) connected to the foot portion (11);
a shank portion (14) connected to the ankle portion (12);
a substrate (17) surrounding the wearing space and extending from the foot portion (11) to the shank portion (14) through the ankle portion (12); and an elastomer (18) disposed adjacent to the substrate (17) and surrounding the wearing space, and when in use, extending from the instep (112) of the foot portion (11) at a cuneiform bone (E) of a wearer (30) through the arch (113) and the sole (111) in order, wrapping over a cuboid bone (A) of the wearer (30), extending through the ankle portion (12) inwardly and upwardly toward the shank portion (14), and wrapping over a talus bone (C) of the wearer (30); and
**characterized in that**
each one of the two bodies (10c) has
a knee portion (15) connected to the shank portion (14); and
a thigh portion (16) connected to the knee portion (15); and
the high-arched foot orthosis has a pelvis portion (20) connected to the two thigh portions (16) of the two bodies (10c), and when in use, wrapping over a hip joint (H) of the wearer (30);
wherein the elastomer (18) of each one of the two bodies (10c) extends through a rear side of the shank portion (14), obliquely extends in combination with upward and outward turns through an outer side of the knee portion (15), spirally extends through the thigh portion (16) and the pelvis portion (20), intersects with the elastomer (18) of the other one of the two bodies (10c) at a front side of the pelvis portion (20), and when in use, surrounds and fetters where the wearer's pelvis and the wearer's waist meet.

## Patentansprüche

1. Hohlfußschuheinlage, die zur Anbringung an den unteren Gliedmaßen eines menschlichen Körpers geeignet ist und Folgendes umfasst:
zwei Körper (10b), wobei jeder der beiden Körper (10b) Folgendes aufweist
einen im Inneren des Körpers (10b) gebildeten Trageraum;
ein Fußteil (11) mit
einer Sohle (111), die sich an einer Unterseite des Fußteils (11) befindet;
einen Rist (112), der sich auf einer Oberseite des Fußteils (11) befindet;
ein Gewölbe (113), das sich an einer der beiden vertikalen Seiten des Fußteils (11) befindet, dem anderen der beiden Körper (10b) zugewandt ist und mit der Sohle (111) und dem Rist (112) verbunden ist; und
eine seitliche Seite (114), die sich an der anderen der beiden vertikalen Seiten des Fußteils (11) befindet und mit der Sohle (111) und dem Rist (112) verbunden ist;
ein Knöchelteil (12), das mit dem Fußteil (11) verbunden ist;
ein mit dem Knöchelteil (12) verbundenes Schaftteil (14);
ein Substrat (17), das den Trageraum umgibt und sich von dem Fußteil (11) zu dem Schaftteil (14) durch das Knöchelteil (12) erstreckt; und ein Elastomer (18), das angrenzend an das Substrat (17) angeordnet ist und den Trageraum umgibt und sich im Gebrauch vom Rist (112) des Fußteils (11) an einem Keilbein (E) eines Trägers (30) durch das Gewölbe (113) und die Sohle (111) erstreckt und nacheinander ein Würfelbein (A) des Trägers (30) umhüllt, sich durch das Knöchelteil (12) nach innen und nach oben zum Schaftteil (14) erstreckt und einen Talusknochen (C) des Trägers (30) umhüllt; und **dadurch gekennzeichnet, dass**
jeder der beiden Körper (10b)
ein Knieteil (15) aufweist, das mit dem Schaftteil (14) verbunden ist; und
ein Oberschenkelteil (16), das mit dem Knieteil (15) verbunden ist;
wobei sich das Elastomer (18) jedes der beiden Körper (10b) durch eine Rückseite des Schaftteils (14) zum Oberschenkelteil (16) erstreckt, sich schräg in Kombination mit Aufwärts- und Auswärtsdrehungen durch eine Außenseite des Knieteils (15) zu einer Vorderseite des Oberschenkelteils (16) erstreckt und das Oberschenkelteil (16) umgibt und fesselt.

2. Hohlfußschuheinlage, die zur Anbringung an den unteren Gliedmaßen eines menschlichen Körpers geeignet ist und Folgendes umfasst:
zwei Körper (10c), wobei jeder der beiden Körper (10c) Folgendes aufweist
einen im Inneren des Körpers (10c) gebildeten Trageraum;
ein Fußteil (11) mit
einer Sohle (111), die sich an einer Unterseite des Fußteils (11) befindet;
einen Rist (112), der sich auf einer Oberseite des Fußteils (11) befindet;
ein Gewölbe (113), das sich an einer der beiden vertikalen Seiten des Fußteils (11) befindet, dem anderen der beiden Körper (10c) zugewandt ist und mit der Sohle (111) und dem Rist (112) verbunden ist; und
eine seitliche Seite (114), die sich an der anderen der beiden vertikalen Seiten des Fußteils (11) befindet und mit der Sohle (111) und dem Rist (112) verbunden ist;
ein Knöchelteil (12), das mit dem Fußteil (11) verbunden ist;
ein mit dem Knöchelteil (12) verbundenes Schaftteil (14);
ein Substrat (17), das den Trageraum umgibt und sich vom Fußteil (11) zum Schaftteil (14) durch das Knöchelteil (12) erstreckt; und
ein Elastomer (18), das angrenzend an das Substrat (17) angeordnet ist und den Trageraum umgibt und sich im Gebrauch vom Rist (112) des Fußteils (11) an einem Keilbein (E) eines Trägers (30) durch das Gewölbe (113) und die Sohle (111) erstreckt, nacheinander ein Würfelbein (A) des Trägers (30) umhüllt, sich durch das Knöchelteil (12) nach innen und nach oben zum Schaftteil (14) erstreckt und einen Talusknochen (C) des Trägers (30) umhüllt; und **dadurch gekennzeichnet, dass**
jeder der beiden Körper (10c)
ein Knieteil (15) aufweist, das mit dem Schaftteil (14) verbunden ist; und
ein Oberschenkelteil (16), das mit dem Knieteil (15) verbunden ist; und
die Hohlfußschuheinlage ein Beckenteil (20) aufweist, das mit den beiden Oberschenkelteilen (16) der beiden Körper (10c) verbunden ist und im Gebrauch ein Hüftgelenk (H) des Trägers (30) umhüllt;
wobei sich das Elastomer (18) jedes der beiden Körper (10c) durch eine Rückseite des Schaftteils (14) erstreckt, sich schräg in Kombination mit Aufwärts- und Auswärtsdrehungen durch eine Außenseite des Knieteils (15) erstreckt, sich spiralförmig durch das Oberschenkelteil (16) und das Beckenteil (20) erstreckt, sich mit dem Elastomer (18) des anderen der beiden Körper (10c) an einer Vorderseite des Beckenteils (20) schneidet und im Gebrauch die Stelle umgibt und fesselt, an der das Becken des Trägers und die Taille des Trägers zusammentreffen.

## Revendications

1. Orthèse de pied fortement arquée conçue pour être appliquée sur les membres inférieurs d'un corps humain, et comprenant :
deux corps (10b), chacun des deux corps (10b) ayant
un espace de port formé à l'intérieur du corps (10b) ;
une partie pied (11) ayant
une semelle (111) située sur une partie inférieure de la partie pied (11) ;
un cou-de-pied (112) situé sur une partie supérieure de la partie pied (11) ;
une voûte plantaire (113) située sur l'un des deux côtés verticaux de la partie pied (11), faisant face à l'autre des deux corps (10b), et reliée à la semelle (111) et au cou-de-pied (112) ; et
un côté latéral (114) situé sur l'autre des deux côtés verticaux de la partie pied (11), et relié à la semelle (111) et au cou-de-pied (112) ;
une partie cheville (12) reliée à la partie pied (11) ;
une partie jambe (14) reliée à la partie cheville (12) ;
un substrat (17) entourant l'espace de port et s'étendant de la partie pied (11) à la partie jambe (14) en passant par la partie cheville (12) ; et un élastomère (18) disposé de manière adjacente au substrat (17) et entourant l'espace de port, et, lorsqu'il est utilisé, s'étendant du cou-de-pied (112) de la partie pied (11) au niveau d'un os cunéiforme (E) d'un patient (30) en passant par la voûte plantaire (113) et la semelle (111) dans l'ordre, enveloppant un os cuboïde (A) de l'utilisateur (30), passant par la partie cheville (12) vers l'intérieur et vers le haut en direction de la partie jambe (14), et enveloppant le talus (C) du patient (30) ; et **caractérisée en ce que**
chacun des deux corps (10b) a
une partie genou (15) reliée à la partie jambe (14) ; et
une partie cuisse (16) reliée à la partie genou (15) ;
dans laquelle l'élastomère (18) de chacun des deux corps (10b) s'étend vers la partie cuisse (16) en passant par un côté arrière de la partie jambe (14), s'étend obliquement en combinaison avec des tours vers le haut et vers l'extérieur en passant par un côté extérieur de la partie genou (15) vers un côté avant de la partie cuisse (16), et entoure et retient la partie cuisse (16).

2. Orthèse de pied fortement arquée conçue pour être appliquée sur les membres inférieurs d'un corps humain, et comprenant :
deux corps (10c), chacun des deux corps (10c) ayant
un espace de port formé à l'intérieur du corps (10c) ;
une partie pied (11) ayant
une semelle (111) située sur une partie inférieure de la partie pied (11) ;
un cou-de-pied (112) situé sur une partie supérieure de la partie pied (11) ;
une voûte plantaire (113) située sur l'un des deux côtés verticaux de la partie pied (11), faisant face à l'autre des deux corps (10c), et reliée à la semelle (111) et au cou-de-pied (112) ; et
un côté latéral (114) situé sur l'autre des deux côtés verticaux de la partie pied (11), et relié à la semelle (111) et au cou-de-pied (112) ;
une partie cheville (12) reliée à la partie pied (11) ;
une partie jambe (14) reliée à la partie cheville (12) ;
un substrat (17) entourant l'espace de port et s'étendant de la partie pied (11) à la partie jambe (14) en passant par la partie cheville (12) ; et
un élastomère (18) disposé de manière adjacente au substrat (17) et entourant l'espace de port, et lorsqu'il est utilisé, s'étendant depuis le cou-de-pied (112) de la partie de pied (11) au niveau d'un os cunéiforme (E) d'un patient (30) en passant par la voûte plantaire (113) et la semelle (111) dans l'ordre, enveloppant un os cuboïde (A) du patient (30), passant par la partie cheville (12) vers l'intérieur et vers le haut en direction de la partie jambe (14), et enveloppant le talus (C) du patient (30) ; et **caractérisée en ce que**
chacun des deux corps (10c) a
une partie genou (15) reliée à la partie jambe (14) ; et
une partie cuisse (16) reliée à la partie genou (15) ; et
l'orthèse de pied fortement arquée a une partie bassin (20) reliée aux deux parties cuisse (16) des deux corps (10c), et lorsqu'elle est utilisée, s'enroule sur une articulation de la hanche (H) du patient (30) ;
dans laquelle l'élastomère (18) de chacun des deux corps (10c) passe par un côté arrière de la partie jambe (14), s'étend obliquement en combinaison avec des tours vers le haut et vers l'extérieur en passant par un côté extérieur de la partie genou (15), s'étend en spirale en passant par la partie cuisse (16) et la partie bassin (20), se croise avec l'élastomère (18) de l'autre des deux corps (10c) au niveau d'un côté avant de la partie bassin (20), et lorsqu'il est utilisé, entoure et retient l'endroit où le bassin et la taille du patient se rencontrent.
